# EUROPEAN PATENT APPLICATION

(11) **EP 1 909 240 A1**
(43) Date of publication of application: **09.04.2008**
(21) Application number: 06023925.8
(22) Date of filing: 17.11.2006
(51) Int. Cl.: G07F 11/44, A61J 7/00

(54) **System for integrally controlling automatic tablet packaging apparatus and method thereof**

(30) Priority: 20.09.2006 KR 20060091294
(71) Applicant: JVM Co., Ltd., Daegu 704-170 (KR)
(72) Inventor: Kim, Jun-ho, Dalseo-gu Daegu, 704-730 (KR)
(74) Representative: Grünecker, Kinkeldey, Stockmair & Schwanhäusser Anwaltssozietät

(57) **Abstract**

An integrated control system in which a single system integrally manages and controls plural tablet packaging machines and a method thereof are disclosed. The system includes a manipulation unit inputting a manipulation signal, and a manipulation signal and a remote monitoring signal to assign an tablet packaging machine to be checked, a communication unit transmitting and receiving data to/from the tablet packaging machines, a microprocessor storing a program performing communication with the tablet packaging machines and stocks of the tablets, controlling the transmission and reception of data to/from the communication unit, storing and analyzing the stocks and status information of the tablet packaging machines, and controlling displaying of status of the tablet packaging machines, a display controller controlling displaying of current stocks of the tablet packaging machines and abnormality of tablet packaging machines, and a display displaying stocks of the tablet packaging machines and abnormality of tablet packaging machines.

## Description

### BACKGROUND OF THE INVENTION

### Field of the Invention

The present invention relates to a system for integrally controlling an automatic tablet packaging machine, and more particularly, to an integrated control system in which a single system integrally manages and controls plural automatic tablet packaging machines and a method thereof.

### Description of the Related Art

Generally, an automatic tablet packaging machine accommodates tablets in tablet cassettes according to kinds of the tablets, and automatically discharges the tablets from the tablet cassettes according to a prescription when the prescription of a medical doctor is inputted so as to automatically package the tablets per one dose. The automatic tablet packaging machine includes a plurality of tablet cassettes installed on respective tablet feeders to accommodate various tablets. The tablet cassettes installed to the tablet feeders discharge the prescribed tablets per dose to hoppers through discharging chutes formed in the respective tablet feeders according to a signal of a microprocessor, and the tablets discharged to the hoppers are discharged from the lower side of the hoppers to be packaged into a packaging sheet on which directions of how to take the tablets is printed by a printer per dose.

In Europe, a medicine supplying shop to package only medicines prescribed by a medical doctor is assigned. In the medicine supplying shop, several tens of automatic tablet packaging machines are connected such that the respective automatic tablet packaging machines package the tablets according to the prescriptions inputted from respective hospitals. In the medicine supplying shop of Europe, about 2 or 3 pharmacists manages the entire several tens of the automatic tablet packaging machines. However, it is difficult for the pharmacists to check whether some tablets are not supplied to any automatic tablet packaging machine, whether a print ribbon or the packaging sheet must be supplemented, and/or whether there is an abnormality in the automatic tablet packaging machine.

### SUMMARY OF THE INVENTION

Therefore, the present invention has been made in view of the above and/or other problems occurring when several automatic tablet packaging machines are connected to be collectively managed, and it is an aspect of the present invention to provide a system for integrally managing and controlling a plurality of automatic tablet packaging machines to automatically package tablets by a single system and a method thereof.

It is another aspect of the present invention to provide an integrated control system of an automatic tablet packaging machine for managing the stock of overall tablets and expendable supplies such as printer ribbons, packaging sheets, and the like that are taken into custody in association with a custody managing system for managing the stock of overall tablets and expendable supplies, and a method thereof.

In accordance with the present invention, the above and other objects can be accomplished by the provision of a system for integrally controlling automatic tablet packaging machines, including: a manipulation unit to input a manipulation signal to perform a function, and a manipulation signal .and a remote monitoring signal to assign an automatic tablet packaging machine whose status is checked or to check a detailed status of the assigned automatic tablet packaging machine; a communication unit to transmit and receive data to and from the plural automatic tablet packaging machines for automatic packaging the tablets; a microprocessor to store a program for integrally performing the communication with the automatic tablet packaging machines and stocks of the tablets and the expendable supplies, to control the transmission and reception of the data to and from the communication unit, to store and analyze the stocks and the status information of the respective automatic tablet packaging machines obtained through the communication unit, and to control displaying of the status of a specific automatic tablet packaging machine and operation of a corresponding device according to a result of the analysis; a display controller to control displaying of current stocks of the respective automatic tablet packaging machines and whether any automatic tablet packaging machine is abnormal according to a displaying control signal outputted from the microprocessor; and a display connected to the display controller to display the stocks of the respective automatic tablet packaging machines and whether any automatic tablet packaging machine is abnormal.

The communication unit further has functions of transmitting an abnormality message generated when the automatic tablet packaging machines are abnormal via a wireless line and of transmitting monitoring information about a site status to a user's (a pharmacist's) mobile phone.

The microprocessor stores a program for integrally performing the communication with the automatic tablet packaging machines and stocks of the tablets and the expendable supplies, and includes: an information input module to receive functional information manipulated by the manipulation unit; a packaging machine tablet stock managing module to analyze and manage the tablet stocks of the respective automatic tablet packaging machines transmitted through the communication control module; a prescription information processing module to analyze prescription information transmitted through the communication control module so as to estimate stocks that are expected to remain by kind of the tablets when the packaging of the tablets is completed; an insufficient time estimating module to analyze the stocks by kind of the tablets obtained from the packaging machine tablet stock managing module and to estimate an insufficient time when the respective tablets will be insufficient; an insufficient tablet packaging preventing module to determine whether there is an insufficient tablet in association with the prescription information processing module and the packaging machine tablet stock managing module and to generate a control signal for compulsorily stopping a corresponding automatic tablet packaging machine when there is the insufficient tablet; a control module to control the transmission and reception of the data, the management of the tablet stocks of the automatic tablet packaging machines, and storing and displaying of the data; a print control module connected to the control module to control printing of status information of the tablets; a displaying control module to control displaying whether the automatic tablet packaging machines are abnormal and the stocks of the tablets according to the control of the control module; and a storing control module to control storing of the data and power failure detecting data transmitted from the automatic tablet packaging machines according to the control of the control module.

In order to manage the stocks of the tablets and the expendable supplies in the custody through the communication with the custody managing system for managing the stocks of the tablets and the expendable supplies in the custody, the microprocessor further includes an expendable supplies managing module connected to the control module to manage the expendable supplies such as a printer ribbon, a packaging sheet, and the like; and a custody tablet stock managing module to analyze and manage overall tablet stocks of a custody transmitted through the communication control module.

According to the aspect of the present invention, a pharmacist can check the operating status of the automatic tablet packaging machines at a remote place and the stocks of the tablets and expendable supplies at a remote place.

Moreover, since the automatic tablet packaging machines can be monitored without a worker, a single pharmacist can control plural automatic tablet packaging machines.

### BRIEF DESCRIPTION OF THE DRAWINGS

These and/or other objects and advantages of the present invention will become apparent and more readily appreciated from the following description of the embodiments, taken in conjunction with the accompanying drawings, in which:
FIG. 1 is a schematic view illustrating an integrated control system of automatic tablet packaging machines according to the present invention;
FIG. 2 is a detailed block diagram illustrating the integrated control system of automatic tablet packaging machines according to the present invention;
FIG. 3 is a block diagram illustrating an example of a microprocessor in FIG. 2; and
FIG. 4 is a view illustrating examples of a display employed in the integrated control system of automatic tablet packaging machines according to the present invention.

### DESCRIPTION OF THE PREFERRED EMBODIMENTS

Hereinafter, an apparatus for detecting information of an auxiliary tray and a method thereof according to embodiments of the present invention will be described in detail with reference to the accompanying drawings. In the following description of the present invention, if the detailed description of the already known structure and operation may confuse the subject matter of the present invention, the detailed description thereof will be omitted.

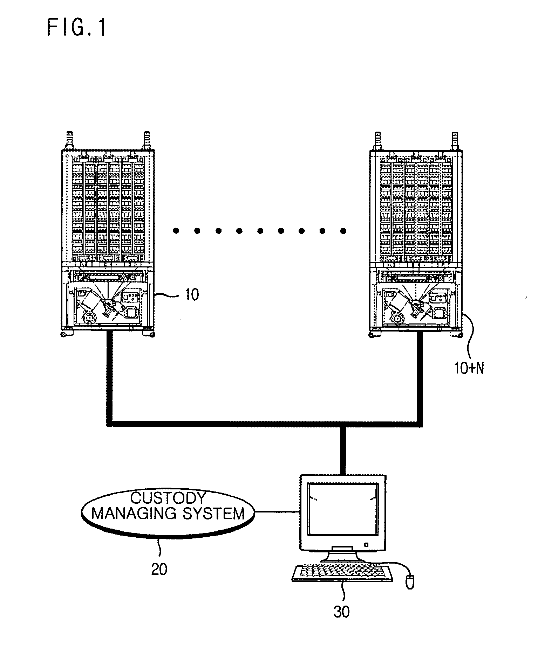

FIG. 1 is a schematic view illustrating an integrated control system of automatic tablet packaging machines according to the present invention.

An integrated control system 30 to integrally control the stocks of tablets and abnormalities of automatic tablet packaging machines 10 to 10+N for automatically packaging the tablets is connected to the automatic tablet packaging machines 10 to 10+N via a local area network (LAN) or a controller area network (CAN). Moreover, the integrated control system 30 may be connected to a custody managing system 20 to manage the tablets and expendable supplies taken into a custody via the LAN or the CAN.

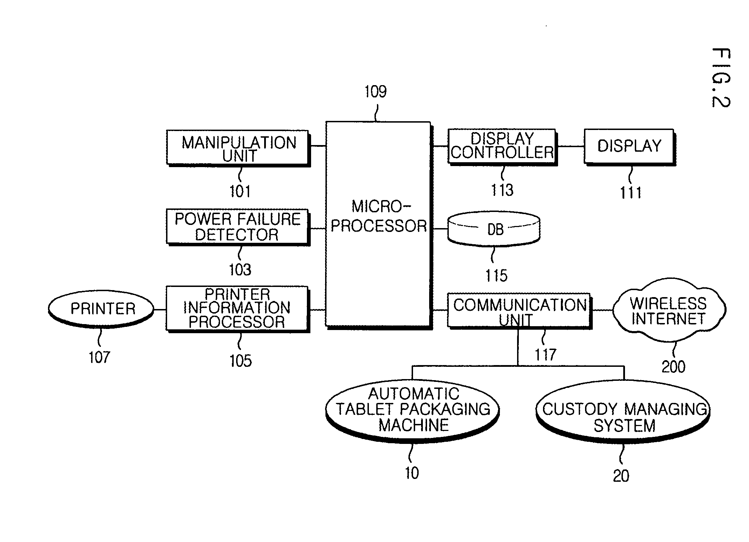

FIG. 2 is a detailed block diagram illustrating the integrated control system of automatic tablet packaging machines according to the present invention.

Here, a manipulation unit 101 inputs a manipulating signal for performing functions, and a manipulating signal for assigning an automatic tablet packaging machine whose status is to be checked or for checking detailed items of the assigned automatic tablet packaging machine, and a power failure detector 103 detects whether power failure occurs in the system. The power failure detector 103 can output an abnormality message when power is cut off.

A print information processor 105 processes print information for outputting information attached to the tablet cassettes of the respective automatic tablet packaging machines, such as tablet information by time, the stock of the tablets, and the like and transmits the print information to a printer 107. A communication unit 117 transmits and receives data to and from the plural automatic tablet packaging machines 10 to 10+N to automatically compound and package the tablets, and preferably LAN, CAN, RS422, and RS485 are used for the communication between the communication unit 117 and the automatic tablet packaging machines 10. In addition, the communication unit 117 is connected to a wireless Internet 200 to transmit an abnormality message and monitoring information about a site status via a wireless line when there is an abnormality in the automatic tablet packaging machines 10 to a mobile phone of a user (a prescription manager). The communication unit 117, moreover, transmits and receives data to and from the custody managing system 20 to manage custody where the tablets and the expendable supplies are obtained via an internal communication.

In addition, a microprocessor 109 stores a program to integrally carry out a communication with the automatic tablet packaging machines 10, the stock of the tablets, and the expendable supplies, controls the transmission and reception of data to and from the communication unit 117, and controls a status displaying of a specific automatic tablet packaging machine 10 and an operation of a corresponding automatic tablet packaging machine 10, according to the stock of the tablets, status information of the respective automatic tablet packaging machines 10 and the result of analysis of the information. In addition, the microprocessor 109 carries out a function of managing the tablets and the expendable supplies in the custody by communicating with the custody managing system 20.

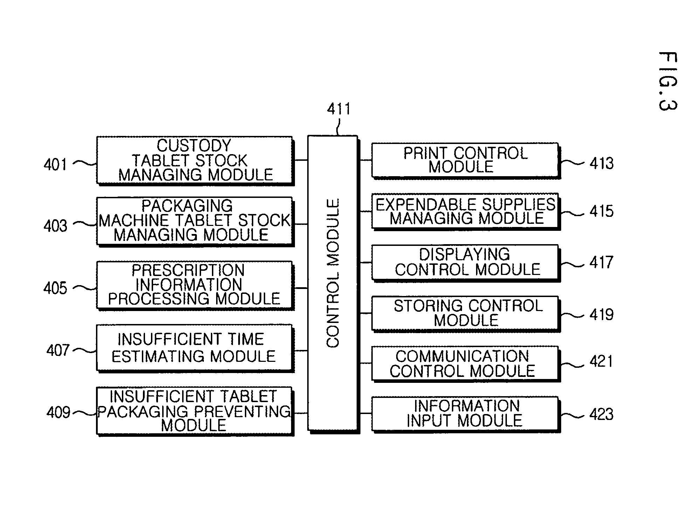

As shown in FIG. 3, the microprocessor 109 includes an information input module 423 to receive functional information manipulated by the manipulation unit 101, a communication control module 421 connected to the communication unit 117 to transmit and receive data to and from the automatic tablet packaging machines 10 and to transmit the abnormality message and site monitoring information to the user's mobile phone via the wireless line and/or a wireless Internet 200, and a packaging machine tablet stock managing module 403 to analyze and manage the stock of the tablets in the respective automatic tablet packaging machines 10 to 10+N, transmitted through the communication control module 421.

Moreover, the microprocessor 109 further includes a prescription information processing module 405 to analyze prescription information transmitted through the communication control module 421 and to estimate the stock, by kind of the tablets, that is expected to remain when the packaging of the tablets is completed, an insufficient time estimating module 407 to analyze the stock by kind of the tablets obtained from the packaging machine tablet stock managing module 403 and to estimate an insufficient time when the respective tablets will be insufficient, and an insufficient tablet packaging preventing module 409 to determine whether there is an insufficient tablet in association with the prescription information processing module and the packaging machine tablet stock managing module and to generate a control signal for compulsorily stopping a corresponding automatic tablet packaging machine when there is an insufficient tablet.

Further, the microprocessor 109 includes a control module 411 to control the transmission and reception of the data, the management of the stock of the tablets in the automatic tablet packaging machines, and the storing and displaying of the data, a print control module 413 connected to the control module 411 to control the printing of status information of the tablets, a displaying control module 417 to control the displaying whether the automatic tablet packaging machines have an abnormality and the stock of the tablets according to the control of the control module 411, and a storing control module 419 to control the storing of data generated from the automatic tablet packaging machines and power failure detecting data according to the control of the control module 411.

Moreover, the microprocessor 109 further includes a custody tablet stock managing module 401 to analyze and manage overall tablet stock of the custody transmitted through the communication control module 421, and an expendable supplies managing module 415 connected to the control module 411 to manage the expendable supplies such as the printer ribbon, the packaging sheet, and the like.

Moreover, a display controller 113 controls the displaying of the stock by kind of the tablets in the respective automatic tablet packaging machines, the stock of the print ribbons, the stock of the packaging sheet, and whether there is an abnormality in the automatic tablet packaging machines and the electric power according to a displaying control signal outputted from the microprocessor 109. The display controller 113 can control the displaying an integrated stock in association with the custody managing system. A display 111 is connected to the display controller 113 to display the stock and the abnormality of the respective automatic tablet packaging machines and overall stock of the custody, effectively and correctly.

Here, when there is an abnormality in the automatic tablet packaging machines distinguished by numbers, the display 111 displays the abnormality by changing a green color indicating a corresponding automatic tablet packaging machine into orange color and red color according to a degree of the abnormality and urgency status. At that time, an alarming lamp installed on the upper side of the corresponding automatic tablet packaging machine is turned on simultaneously with generating an alarm warning sound and an alarming message is displayed on the display. Moreover, when a specific automatic tablet packaging machine is selected, the display 111 can display the stock of the tablets, and the remaining quantity of the print ribbons and the packaging sheet in the selected automatic tablet packaging machine. When the custody managing system is selected, the display 111 preferably displays overall stock of the tablets and the status of the expendable supplies. Even when there is the power failure or an abnormality in the automatic tablet packaging machines, the display 111 displays an alarm warning message.

The integrated control system of the automatic tablet packaging machines according to the present invention and a method thereof will be described in detail as follows.

When the plural automatic tablet packaging machines 10 to 10+n are connected to the custody managing system 20 by LAN, CAN, RS422, or RS485, the automatic tablet packaging machines 10 to 10+N check own stock of the tablets and whether there is an abnormality and transmits the same to the custody managing system 20, periodically.

In more detail, the microprocessor 109 receives various information, such as power failure detecting information, generated when operating the system through the information input module 423, and stores the same in a database 115. The microprocessor 109 analyzes the same and controls the displaying control module 417 to output an abnormality message when a power failure is determined according to a result of the analysis. The displaying control module 417 controls the display controller 113 to display the power failure abnormality message so that the display 111 displays the power failure abnormality message.

Next, when the stock of the tablets remaining in the respective tablet cassettes, the remaining quantity of the print ribbons detected by a sensor installed in the printer, the remaining quantity of the packaging sheet detected by a sensor installed in a sealing device, and the abnormality of the automatic tablet packaging machines are received from the automatic tablet packaging machines via a wired line or through the communication control module 421, a packaging machine tablet stock managing module 403 analyzes information of the respective automatic tablet packaging machines in real time, and the microprocessor 109 determines the stocks of the tablets and whether there is an abnormality then transmits the result of the determination to the control module 411. For example, if number 1 automatic tablet packaging machine has tablets A, B, C, D, E, F, G, and H, the remainder of an initial quantity of tablets less the quantity of the tablets discharged from the tablet cassettes according to the prescription is the current stock of the tablets in the corresponding cartridge. Thus, if a minimal quantity of the tablets is predetermined and stored, whether the tablets are insufficient (must be supplemented) or not can be easily perceived by a message.

In this way, the stocks of the tablets in the respective tablet cassettes of the automatic tablet packaging machines are analyzed and stored in the database 115. Additionally, since the control module 411 can confirm the stock of the tablets and the abnormality of the corresponding automatic tablet packaging machine in association with the packaging machine tablet stock managing module 403, the display controller 113 controls the display 111 to display the status of the corresponding automatic tablet packaging machine in real time.

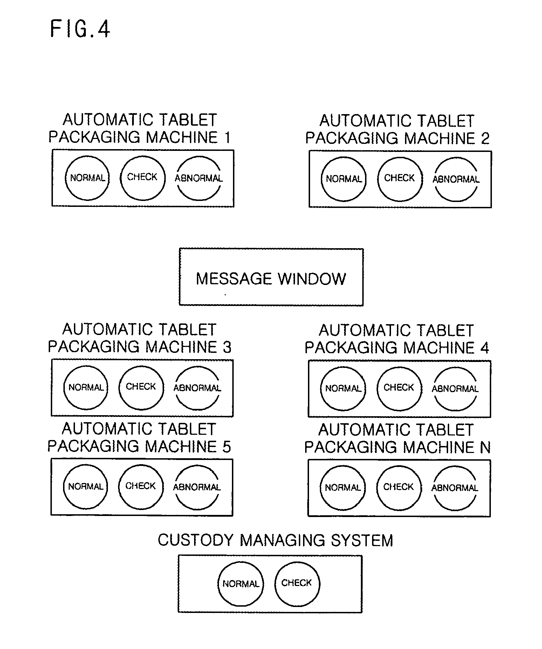

FIG. 4 is a view illustrating examples of the display 111. The display 111 displays information for distinguishing the respective automatic tablet packaging machines, the stocks of the tablets of the respective automatic tablet packaging machines, and the abnormality of the respective automatic tablet packaging machines. Preferably, the display 111 displays the information using light emitting diodes (LED) in which respective LEDs display a normal status, a checking status, and an abnormality status using text and colors. Here, the normal status indicates a status that the automatic tablet packaging machine is normally operated and is indicated by green. The checking status indicates a status that the automatic tablet packaging machines currently have not malfunctioned but there is a possibility of malfunction (it can be known by self-diagnosis) and a specific tablet must be supplemented, and is indicated by yellow. The abnormality status indicates a status that the automatic tablet packaging machines have malfunctioned or the tablets are insufficient, and is indicated by red.

This displaying method has been described as an example, and various modifications of the displaying method will be apparent to those skilled in the art.

Although the user can perceive which automatic tablet packaging machine is abnormal and/or that tablets must be supplemented to which automatic tablet packaging machine when viewing the message displayed on the display 111, the user cannot know what abnormality has occurred in which automatic tablet packaging machine and/or which tablets must be supplemented in detail when simply viewing the status of the automatic tablet packaging machines displayed on the display 111.

Thus, if the user wishes to know the detailed information, the user selects a specific automatic tablet packaging machine through the manipulation unit 101 and inputs information for the status of the selected automatic tablet packaging machine. Then, the control module 411 detects the input and outputs the information that the user wishes to know in association with the storing control module 419 and displays the information on a message window of the display 111. For example, when the user selects the insufficient tablet of the number 1 automatic tablet packaging machine, the name of the insufficient tablet and the current stock of the tablet are displayed on the message window of the display 111. Here, the message window preferably employs a conventional LCD capable of displaying general data, and any display capable of displaying the data with texts and numbers.

Meanwhile, the status of the automatic tablet packaging machines must be modified in real time whenever the prescription is produced. Thus, when the prescription is transmitted to a specific automatic tablet packaging machine, the prescription information processing module 405 processes the prescription information and compares the information about the tablets in the prescription with the current stock of the tablets in the automatic tablet packaging machine to estimate the stocks by kind of the tablets after completion of the prescription. Needless to say, the estimation and analyzing result thereof are stored in the database 115 in real time.

Moreover, the integrated control system is advantageous for estimating the insufficient time of the respective tablets. In other words, the insufficient time estimating module 407 obtains an average consumption of the tablets by dividing weekly consumptions of the respective tablets by days and compares the average consumption of the tablets with the stocks of the respective tablets currently remaining in the automatic tablet packaging machines to estimate when any tablet will be insufficient. If it is possible to estimate the insufficient time of the tablets, the insufficient time is displayed on the display 111 such that the user perceives a tablet will be insufficient. Thus, the corresponding tablets are bought in advance to prevent the compounding of the prescription from being impossible due to the insufficiency of a specific tablet. Since the buying of the tablets can be planned due to the estimation of the insufficient time, more convenience can be provided to the user.

Meanwhile, the conventional automatic tablet packaging machine 10 has disadvantage to continuously package the tablets until the user stops the operation of the automatic tablet packaging machine even when a specific tablet is insufficient during the automatic compounding and packaging of the tablets according to the prescription. In other words, even when a certain tablet is insufficient during automatically packaging the tablets in response to the prescription, the conventional automatic tablet packaging machine 10 automatically compounds and packages the rest of the tablets.

In order to solve the above problem, in the present invention, when there is an automatic tablet packaging machine in which a specific tablet is insufficient, the insufficient tablet packaging preventing module 409 transmits information to the corresponding automatic tablet packaging machine through the communication control module 421 in association with the control module 411 and the prescription information processing module 405 such that the corresponding automatic tablet packaging machine is stopped. Needless to say, when the automatic tablet packaging machine is automatically stopped, the display 111 displays this status.

In addition, the microprocessor 109 controls the print control module 413 to print the status information required by the user so that the user can confirm the status information and the managing history of the corresponding automatic tablet packaging machine through a document printed by the printer 107.

As described above, the integrated management of the automatic tablet packaging machines does not matter when the user visually continuously checks the display, however, on occasion the user may be away from the integrated control system 30 for a long time.

In this case, a rapid necessary means may be required to deal with and unexpected abnormality. To this end, in the present invention, when any necessary means is not taken within a predetermined time (for example, ten minutes) although the microprocessor 109 controls the display 111 to display whether a specific automatic tablet packaging machine 10 is abnormal or not and/or the insufficient tablet, the microprocessor 109 generates an abnormality message and transmits the abnormality message to the mobile phone of the user through the communication control module 421. Thus, the communication unit 117 transmits the generated abnormality message to the user's mobile phone via the wireless Internet 200.

By doing so, the user far away from the integrated control system 30 can easily perceive the abnormality status of a specific automatic tablet packaging machine so that the user can rapidly take a necessary means to prevent the packaging of the tablets from being stopped for a long time and various problems from occurring due to the malfunction.

Moreover, in order for the integrated control at a place where the user cannot confirm the display 111, in the present invention, the system monitoring using the wireless Internet 200 is possible. In other words, when a manipulation signal for a remote monitoring is inputted by the manipulation unit 101, the microprocessor 109 stores the manipulation signal in the database 115 and switches to a mode for remote system monitoring. The site statuses of the respective automatic tablet packaging machines are captured by a camera and are transmitted to a mobile phone assigned by the user through the wireless Internet 200 periodically or occasionally. Thus, the user can monitor the site status using the mobile phone at a remote place. Here, although not depicted in the drawings, the camera is necessary for the site monitoring, and since function and operation of the camera are conventional, the detailed description will be omitted.

Meanwhile, the integrated control system 30 according to the present invention can manage overall stocks of the tablets and the stocks of the expendable supplies in custody in association with the custody managing system 20 to manage the custody in which the tablets and the expendable supplies are stored.

In other words, when the integrated control system 30 is connected to the custody managing system 20 through LAN or CAN, the custody managing system 20 transmits the stock status of the overall tablets and status information of the expendable supplies such as print ribbons, packaging sheets, and the like stored in the custody to the integrated control system 20 periodically or occasionally.

The custody tablet stock managing module 401 analyzes the statuses of the overall tablets and the expendable supplies such as print ribbons, packaging sheets, and the like stored in the custody among the received information. The analyzed custody information is stored in the database 115 by the storing control module 419.

When, as result of the analysis of the custody information, it is determined that a specific tablet or specific expendable supplies are insufficient, or that the tablet or expendable supplies are not insufficient now but are expected to be sufficient within the near future, this status is informed to the control module 411. The control module 411 controls the storing control module 410 to store the corresponding information and controls the displaying control module 417 to display the insufficient status of the tablet or expendable supplies or the expected time when the corresponding tablet or expendable supplies will be insufficient on the message window of the display 111 (See FIG. 4). Then, the user (the prescription manager) can easily perceive the managing status of the tablets and the expendable supplies of the custody through the display 111 without directly going and checking the custody. After that, a necessary means is taken to prevent the problem that the prescription is not carried out due to the insufficiency of the tablets or the expendable supplies.

As described above, plural automatic tablet packaging machines for automatically compounding and packaging tablets can be integrally managed and controlled by a single system.

Moreover, a single prescription manager can easily control and manage the plural automatic tablet packaging machines for automatically compounding and packaging tablets.

In addition, according to the present invention, whether the respective packaging machines are abnormal or not can be checked by a single display, a detailed status of an abnormal packaging machine can be checked, an abnormality message can be transmitted via a wireless line, and the site status monitoring information can be provided to a user at a remote place through a wireless Internet.

Moreover, according to the present invention, the integrated control system integrally and conveniently manages the tablets and the expendable supplies in a custody by the communication with the custody managing system for managing the stocks of the custody in which the tablets and the expendable supplies are stored.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A system for integrally controlling automatic tablet packaging machines to automatically package tablets according to a prescription, the system comprising:
a manipulation unit to input a manipulation signal to perform a function, and a manipulation signal and a remote monitoring signal to assign an automatic tablet packaging machine whose status is checked or to check a detailed status of the assigned automatic tablet packaging machine;
a communication unit to transmit and receive data through an internal communication with the automatic tablet packaging machines;
a microprocessor to store a program for integrally performing the communication with the automatic tablet packaging machines and stocks of the tablets, to control the transmission and reception of the data to and from the communication unit, to store and analyze the stocks and the status information of the respective automatic tablet packaging machines obtained through the communication unit, and to control displaying of the status of a specific automatic tablet packaging machine and operation of a corresponding device according to a result of the analysis;
a display controller to control displaying of current stocks of the respective automatic tablet packaging machines and whether any automatic tablet packaging machine is abnormal according to a displaying control signal outputted from the microprocessor; and
a display connected to the display controller to display the stocks of the respective automatic tablet packaging machines and whether any automatic tablet packaging machine is abnormal.

2. The system for integrally controlling automatic tablet packaging machines according to claim 1, wherein the communication unit further comprises functions of transmitting an abnormality message generated when the automatic tablet packaging machines are abnormal via a wireless line and of transmitting monitoring information about a site status to a user's mobile phone.

3. The system for integrally controlling automatic tablet packaging machines according to claims 1 or 2, wherein the communication unit is connected to the automatic tablet packaging machines through one of a local area network (LAN), a controller area network (CAN), an RS422, and an RS485 to transmit and receive data and to carry out a wireless communication with a user via a wireless Internet.

4. The system for integrally controlling automatic tablet packaging machines according to claim 1, wherein the microprocessor comprises:
an information input module to receive functional information manipulated by the manipulation unit;
a communication control module connected to the communication unit to transmit and receive the data to and from the automatic tablet packaging machines and to transmit an abnormality message via a wireless communication, and to transmit site monitoring information to a user's mobile phone via a wireless Internet;
a control module to control the transmission and reception of the data, the management of the tablet stocks of the automatic tablet packaging machines, and storing and displaying of the data;
a packaging machine tablet stock managing module to analyze and manage the tablet stocks of the respective automatic tablet packaging machines transmitted through the communication control module;
a displaying control module to control displaying whether the automatic tablet packaging machines are abnormal and the stocks of the tablets according to the control of the control module; and
a storing control module to control storing of the data and power failure detecting data transmitted from the automatic tablet packaging machines according to the control of the control module.

5. The system for integrally controlling automatic tablet packaging machines according to claims 1 or 4, wherein the microprocessor further comprises:
a prescription information processing module to analyze prescription information transmitted through the communication control module so as to estimate stocks that are expected to remain by kind of the tablets when the packaging of the tablets is completed;
an insufficient time estimating module to analyze the stocks by kind of the tablets obtained from the packaging machine tablet stock managing module and to estimate an insufficient time when the respective tablets will be insufficient; and
an insufficient tablet packaging preventing module to determine whether there is an insufficient tablet in association with the prescription information processing module and the packaging machine tablet stock managing module and to generate a control signal for compulsorily stopping a corresponding automatic tablet packaging machine when there is the insufficient tablet.

6. The system for integrally controlling automatic tablet packaging machines according to claims 1 or 4, wherein the microprocessor further comprises:
a print control module connected to the control module to control printing of status information of the tablets;
an expendable supplies managing module connected to the control module to manage the expendable supplies such as a printer ribbon, a packaging sheet, and the like; and
a custody tablet stock managing module to analyze and manage overall tablet stocks of a custody transmitted through the communication control module.

7. The system for integrally controlling automatic tablet packaging machines according to claim 1, further comprising:
a power failure detector to detect whether power failure occurs in the system so as to transmit an abnormality message when power is cut off; and
a print information processor to process print information for outputting status information of respective automatic tablet packaging machines and the stocks of the tablets and to transmit the print information to a printer

8. The system for integrally controlling automatic tablet packaging machines according to claim 1, wherein the display displays information to distinguish the respective automatic tablet packaging machines, the tablet stocks of the respective automatic tablet packaging machines, and whether the automatic tablet packaging machines are abnormal using light emitting diode, and respective light emitting diodes display a normal status, a checking status, and an abnormality status using text and colors.

9. The system for integrally controlling automatic tablet packaging machines according to claim 8, wherein the normal status indicates a status, indicated by a green colored light emitting diode, that the automatic tablet packaging machines are normally operated and the stocks of the tablets by respective cartridges are sufficient, the checking status indicates a status, indicated by a yellow colored light emitting diode, that the automatic tablet packaging machines currently have not malfunctioned but there is a possibility of malfunction or a specific tablet must be supplemented, and the abnormality status indicates a status, indicated by a red colored light emitting diode, that the automatic tablet packaging machines have malfunctioned or the tablets are insufficient.

10. The system for integrally controlling automatic tablet packaging according to claim 5, wherein the insufficient time estimating module obtains an average consumption of the tablets by dividing weekly consumptions of the respective tablets by days and compares the average consumption of the tablets with the stocks of the respective tablets currently remaining in the automatic tablet packaging machines to estimate when any tablet will be insufficient.

11. A method of integrally managing automatic tablet packaging machines and a custody managing system using a system comprising: a manipulation unit to input a manipulation signal to perform a function; a communication unit to transmit and receive data through an internal communication with the automatic tablet packaging machines and the custody managing system; a microprocessor to store a program for integrally performing the communication with the automatic tablet packaging machines and the custody managing system, stocks of the tablets, and expendable supplies, and to control overall operation of the system; a display controller to control displaying of current stocks of the respective automatic tablet packaging machines, whether any automatic tablet packaging machine is abnormal, and overall stocks of the tablets and the expendable supplies in the custody; and a display to display the current stocks of the respective automatic tablet packaging machines, whether any automatic tablet packaging machine is abnormal, and the overall stocks of the tablets and the expendable supplies in the custody, the microprocessor carrying out:
receiving functional information manipulated by the manipulation unit;
transmitting and receiving data to and from the automatic tablet packaging machines and the custody managing system and transmitting an abnormality message via a wireless line and site monitoring information to a mobile phone via a wireless Internet when being connected to the communication unit;
controlling the transmission and reception of the data, management of the stocks of the tablets in the custody and the automatic tablet packaging machines, and storing and displaying of the data;
analyzing and managing the overall stocks of the tablets in the custody;
analyzing and managing the overall stocks of the tablets in the respective automatic tablet packaging machines;
analyzing prescription information to estimate the stocks that are expected to remain by kind of the tablets when packaging of the tablets is completed;
analyzing the stocks by kind of the tablets to estimate an insufficient time when the respective tablets will be insufficient;
determine whether there is an insufficient tablet and generate a control signal for compulsorily stopping a corresponding automatic tablet packaging machine when there is an insufficient tablet;
controlling printing of status information of the tablets;
analyzing information of the custody managing system to manage the expendable supplies such as printer ribbons, packaging sheets, and the like;
controlling displaying whether the automatic tablet packaging machines are abnormal and of the stocks by kind of the tablets in the respective automatic tablet packaging machines; and
controlling storing of the data transmitted from the automatic tablet packaging machines and the custody managing system and power failure detecting data.
